# EUROPEAN PATENT SPECIFICATION

(11) **EP 4 437 960 B1**
(45) Date of publication and mention of the grant of the patent: **24.12.2025**
(21) Application number: 24161152.4
(22) Date of filing: 04.03.2024
(51) Int. Cl.: A61B 5/154, A61B 5/15

(54) **IMPROVED MULTI-SAMPLE BLOOD COLLECTION HYPODERMIC NEEDLE**
VERBESSERTE HYPODERMISCHE NADEL ZUR BLUTENTNAHME AUS MEHREREN PROBEN
AIGUILLE HYPODERMIQUE DE PRÉLÈVEMENT SANGUIN MULTI-ÉCHANTILLONS AMÉLIORÉE

(30) Priority: 28.03.2023 IT 202300005964
(43) Date of publication of application: 02.10.2024
(73) Proprietor: Sol-Millennium Swiss R&D Center SA, 6900 Lugano (CH)
(72) Inventor: LIN, Liang, Chicago, IL 60611 (US); DE ZOLT, Dario, 21054 Fagnano Olona (VA) (IT); BELLUZZI, Marco, 22100 Como (CO) (IT); MASPERO, Andrea, 21040 Venegono Inferiore (VA) (IT); LAGANA', Matteo, 22030 Longone al Segrino (CO) (IT)
(74) Representative: Ripamonti, Enrico

(56) References cited:
- EP-A1- 0 478 459
- EP-A1- 0 619 096
- WO-A1-2015/076221
- JP-A- 2002 325 749
- US-A1- 2002 161 336

## Description

The present invention relates to a hypodermic needle for multi-sample blood collection according to the preamble of claim 1. Such a hypodermic needle is known from EP0478459.

A multi-sample blood collection needle comprises a needle having two portions which are opposite and associated with a support element of the needle or hub. The two opposite portions of the aforementioned needle protrude from opposite sides of the latter.

The opposite portions of the needle have a sharp tip each. A first portion is adapted to be inserted into a vein of the patient, while the second portion is covered by an elastic covering element; such second portion is adapted to cooperate with blood collection member (such as a test tube, for example) which - when coupled to the second portion of the needle - brings the elastic element in contact with the corresponding sharp tip which pierces it. Continuing the coupling of the collection member with such second portion of the needle (which penetrates into a usual element or cap for closing said collection member), the collapsible element is folded towards the support element or hub (pushed by such closing element of said member) and the blood may therefore flow into the collection member.

As known, when the sharp tip of the first portion of the needle pierces a vein of a patient, the blood pressure (slightly higher than the atmospheric one) pushes the air contained in a cavity of the needle (which connects the two end sharp tips); the air exits from the tip of the second portion in the collapsible covering element. In order to discharge such air from the needle, there is provided for a space between the second portion of the needle and the support element connected to an air vent hole obtained in the support element.

Given that together with air, in the cavity of the needle and in the space between the latter and the support element, there also moves the blood, along the path in the aforementioned space (parallel to the longitudinal axis of the needle) or in proximity or on the vent hole there is provided for a ventilation filter. Such filter may be of the "self-sealing" type that is it has a porosity which shuts automatically when it is in contact with blood, thanks to an appropriate additive which gels occluding the pores, therefore preventing exit from the ventilation hole.

As mentioned, the ventilation filter may be arranged in a special chamber like EP3038534 or EP2097123 made in proximity of the ventilation hole. Alternatively, such ventilation filter may be arranged around the needle and in the space between the needle and the support element of the needle as described in EP3046472; or, the aforementioned filter may be arranged perpendicularly and to the axis of a channel which connects the space around the aforementioned needle and the hole which opens laterally to the support element, for example as described in EP0478459.

When the blood moves in the filter chamber or in a channel which connects the space between the needle and the hub of the needle with the vent hole, such movement occurs in the support element or hub of the needle; it is usually transparent and it therefore acts as a reflux chamber which allows to visually detect whether the vein has been pierced.

The prior art solutions for allowing the outflow of the air from said support element of the needle reveal some drawbacks. For example, providing for the ventilation filter coaxial to the needle and between the latter and the support element (acting as a reflux chamber) could limit the use of large-sized needles (for example with diameter measuring 18G: as a matter of fact such use could lead to an excessive size of the support element of the needle, which could lead to the device not being compatible with the usual supports of threaded test tubes available on the market. Alternatively, so as not to widen the support element of the needle, the required changes could lead to wall thicknesses of the components which are so small to an extent of being difficult to manufacture or provide the filter with thickness that is too thin to allow an appropriate ventilation of the reflux chamber.

The presence of channels in the support element and which open laterally thereto (that is through a hole having a longitudinal axis perpendicular to the cavity of the needle) may entail that, in case of the ventilation filter made or assembled with defects or damaged, the blood flows out with the air laterally to the support element with obvious drawbacks for the healthcare operators who are using the needle.

According to the present invention there is provided a hypodermic needle comprising the features of claim 1. Preferred embodiments of the present invention are defined in the dependent claims.

An object of the present invention is to provide a multi-sample blood collection hypodermic needle which is improved with respect to the equivalent needles of the prior art.

In particular, an object of the present invention is to provide a hypodermic needle of the type mentioned above which ensures that the blood drops that might possibly follow the air flow towards the ventilation hole do not exit immediately towards an area of potential contact with the operator.

Another object is to provide a hypodermic needle of the type mentioned above which allows the use of needles with large cross-section, such as needles with size equal to or greater than 18G.

A further object is to provide a hypodermic needle of the type mentioned above which allows a rapid time for preparation for use.

Another object is to provide a hypodermic needle of the type mentioned above which has at least one part thereof adapted to allow the quick identification of the size thereof.

A further object is to provide a hypodermic needle of the type mentioned above which has a support element or reflux chamber which allows a quick and simple identification of a correct insertion of the needle into the vein.

These and other objects which shall be more apparent to the person skilled in the art are attained by a hypodermic needle according to the attached claims.

For a better understanding of the present invention hereto attached are the following drawings, wherein:
figure 1 shows an exploded view of a hypodermic needle according to the invention prior to use;
figure 2 shows a perspective view - from one side - of a hypodermic needle according to the invention;
figure 3 shows an exploded view - from a first angle - of a part of the hypodermic needle of figure 1;
figure 4 shows a view similar to that of figure 3, but taken from another angle;
figure 5 shows the hypodermic needle according to the invention coupled to an element for containing a blood collection member such as a test tube;
figure 6 shows an enlarged view of a detail of the hypodermic needle of figure 1 coupled to the containment element of figure 5;
figure 7 shows a cross-sectional view according to line 7-7 of figure 6;
figure 8 shows a partial longitudinal cross-sectional view of the hypodermic needle coupled to the containment element of figure 5 and showing the path of the air contained in the needle upon the first use thereof;
figure 9 shows a perspective view of the hypodermic needle of figure 1 coupled with a safety device for protection against accidental contact with the needle after use; and
figure 10 shows a bottom perspective view of part of the hypodermic needle and of the safety device according to figure 9.

With reference to the aforementioned figures, a hypodermic needle for multi-sample blood collection through the vein is indicated generally with reference numeral 1 and it comprises a hub or an element for supporting the needle 2 from whose opposite ends 2A, 2B opposite portions 3 and 4 of a needle 5 having sharp tips 3A and 4A, respectively protrude. Internally, the needle 5 has a continuous channel or cavity 6 which connects the tips 3A and 4A so as to transfer the collected blood from a vein of a patient from a first tip 3A to the second tip 4A.

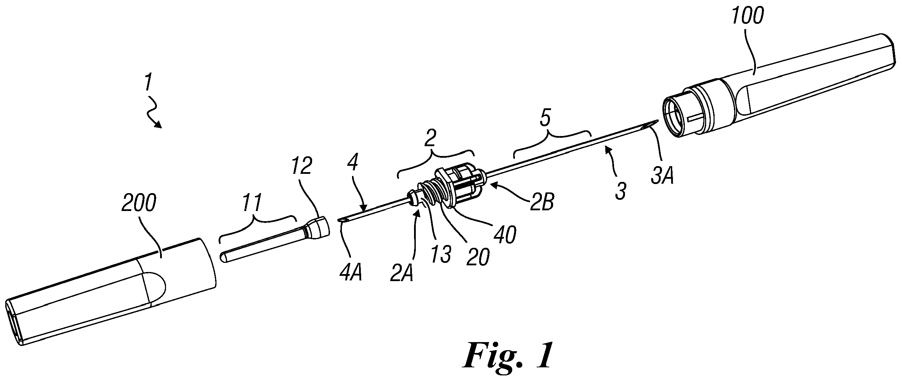

In figure 1, the hypodermic needle 1 is associated with protection caps 100 e 200 arranged on the portions 3 and 4 of the needle 5.

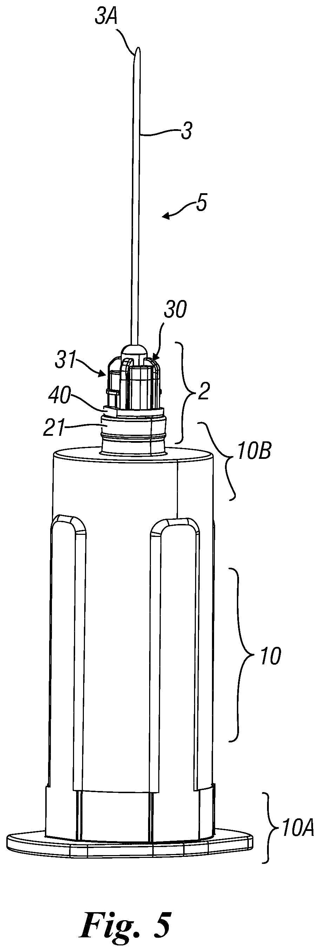

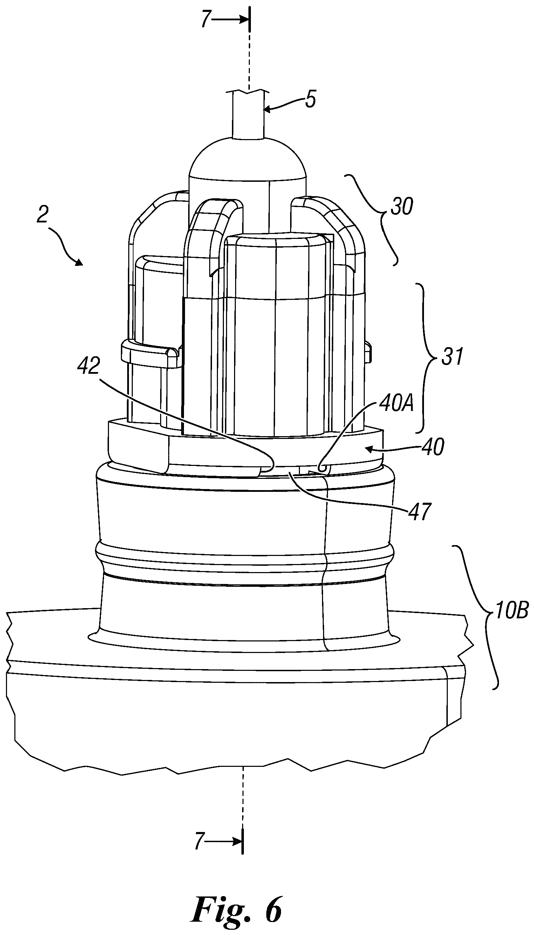

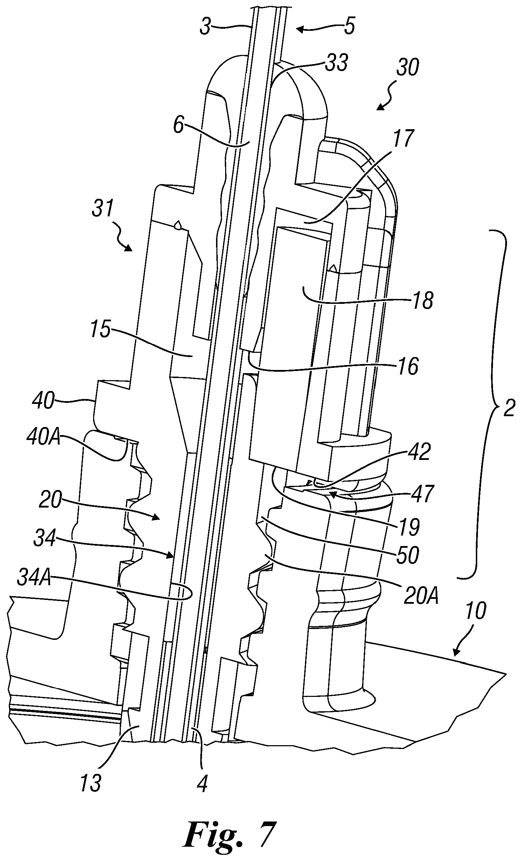

The first portion 3 of the needle is adapted to be inserted into the vein of the patient, while the second portion 4 is adapted to transfer the collected blood to a collection member, such as a test tube (not shown). Around said second portion, integrally joined with the hub 2, a hollow cylindrical body 10 (see figures 5-8) adapted to contain the test tube during the filling thereof with blood is adapted to be arranged in such case. Such hollow cylindrical body 10 is open at one end 10A and it has the opposite end 10B adapted to cooperate with the hypodermic needle 1 (in particular with the hub 2).

The second portion 4 of the needle is inserted into the covering element 11 which is substantially cylindrical and integrally joined with said element for supporting the needle or hub 2 given that it is elastically fitted, with one end 12 thereof, on a protruding part 13 protruding from the end 2A of said element for supporting the needle.

The covering element 11 is made of collapsible but elastic material and it is adapted to be pushed towards the aforementioned support element or hub when the needle 5 is associated with the blood collection member such as the test tube.

The element for supporting the needle or hub 2 is made of transparent material and it allows to see the blood flowing through it. Therefore, such hub acts as a reflux chamber and for displaying the blood during the collection.

When a usual test tube, provided with cap, is brought into contact with the cylindrical element 11 mentioned above and it is pushed on the second portion 4 of the needle, the tip 4A of the latter penetrates into the test tube and the cylindrical element 11 is pierced and therefore pushed towards the hub. All this in a per se known manner.

Between the needle 5 and the hub or support element of the needle 2 there is present a passage or space 15 which communicates with a channel 16 obtained in the support element or hub 2 (hereinafter solely referred to as "hub 2") and which terminates in a chamber 17 in which there is arranged a filtering element or ventilation filter 18 (made of per se known self-sealing porous material such as HDPE). The chamber 17 of the filter is therefore open towards the external of the hub 2 through a vent hole 19 present in an end of the chamber 17.

Such passage allows the air, which is pushed by the blood when it penetrates into the needle and present in the latter, to flow out from the hypodermic needle 5 and from the covering element 11 of the second portion 4 (before it is pierced) through said space 15 and said filter 18 up to the external of the hub 2. Any blood which flows through the cavity of the needle together with the air it may also reach the chamber 17 of the filter, but it is blocked by the latter.

All this in a known manner.

In a first aspect, the chamber 17 for the ventilation filter 18 is arranged, in the embodiment of the figures, with longitudinal axis K parallel to a longitudinal axis W of the needle 5 and it is however present only on one side of the needle. The filter is of the per se known type and it is present in said chamber 17 of the filter up to the vent hole 19, the latter having a median axis (which, in the embodiment of the figures, is superimposed to the axis K) parallel to the longitudinal axis of the needle 5.

According to another aspect, the vent hole 19 opens to the external at a threaded portion (externally) 20 of the hub 2 adapted to be engaged (by screwing) with a corresponding (internally) threaded part 21 of the end 10B of the hollow cylindrical body 10 mentioned further above. Such engagement constrains the hypodermic needle 1 to said hollow cylindrical body 10. Other connection mechanisms, such as snap-coupling fittings, bayonet coupling and the like are also possible. Therefore, the hub 2 generally has a constraint portion 20 adapted to be coupled with a corresponding constraint part 21 of the hollow cylindrical body 10, said coupling being of the mechanical type or by threading or with a snap-coupling, bayonet coupling or the like.

In particular, the vent hole 19 opens towards the hollow cylindrical body 10 or towards the threaded portion 20 of the hub 2 (or equivalent part for constraining the hub to the body 10). Therefore, any loss of blood that may flow out from the vent hole 19 would follow the threading 20A of the threaded portion 20, or small channels that will be referred to as "discharge" channels designed for this purpose (in case of connection other than the threaded one) and it would not spill on the side of the hub 2. The principle of operation of this fluidic trap (defined by "discharge channels", whether defined between the engaged threads of the portion 20 and of the part 21 or by the channels present between such portions and parts) is based on a preferential path guided by capillary forces (the system has an energy gain due to the adhesion forces) which prevent the immediate formation of a lateral drop whose size and mass exceeds the cohesion thereof, due to the surface tension, and therefore fall from the side of the hub 2.

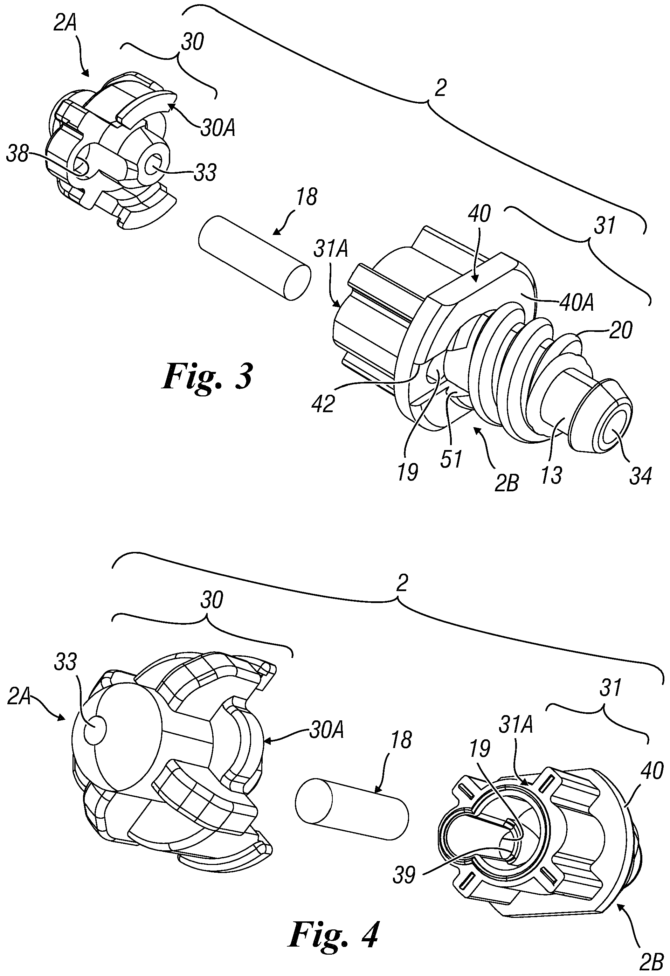

The support element of the needle or hub 2 comprises a first portion 30 and a second portion 31 shown in exploded view in figures 3 and 4. Such portions may be both or only partly coloured to indicate the size of the needle. Alternatively, such colouring may be applied to the (both or individually) caps 100 and 200. Such colouring may comply with the ISO 6009 standard. Such portions 30 and 31 are constrained to each other in any known manner: for example, end parts thereof, respectively 30A and 31A adapted to be arranged facing each other, are heat sealed or ultrasonic sealed so as to integrally join them with and define the hub 2 as if it were a single body.

The first portion 30 has and defines the first end 2A of the hub, while the second portion 31 has and defines the second end 2B of the hub. The first portion 30 has a through hole 33 which contains the needle 5 and from which the first portion 3 of the needle protrudes. In such through hole 33, which connects the first end 2A of the hub and the end portion (internal, with reference to the hub 2) 30A of the first portion 30, the needle is fixed in any known manner (usually by gluing).

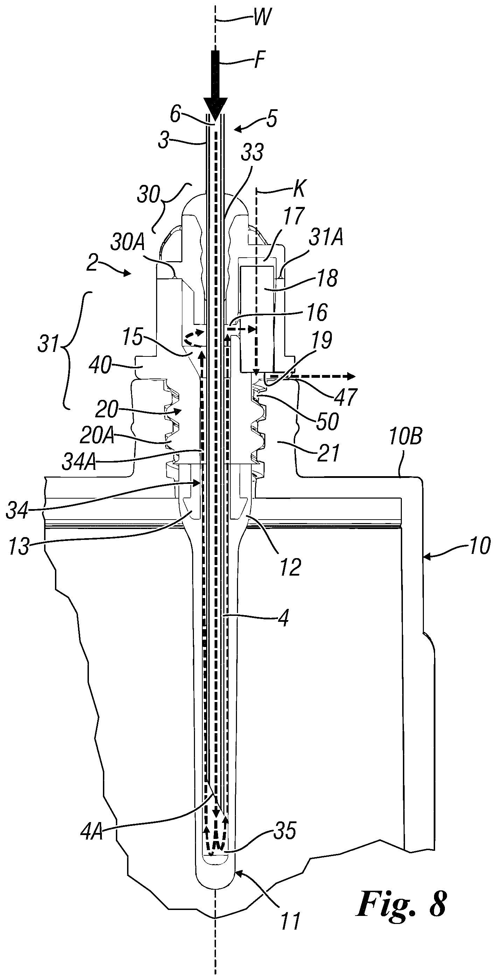

The second portion 31 of the hub, instead has a through hole 34 connected to the space or passage 15, said through hole being cross-sectionally larger than the diameter of the needle 5. Therefore, the through hole 34 is also connected with an internal volume 35 of the covering element 11 of the second portion 4 of the needle 5 so as to receive - from said volume 35 - the air coming from the channel or cavity 6 of the needle 5 (pushed by the blood after the needle has been inserted into the vein); therefore, such air flows (see arrow F of figure 8 and the dashed line shown in such figure) through the through hole 34 in the vacant space defined by the distance between the wall 34A of the through hole and the needle, reaches the space or passage 15 and from here to the chamber 17 of the filter. Obviously, the through hole 34 may be directly connected with the channel 16 and the chamber 17 of the filter.

The first and the second portion 30 and 31 of the hub 2 comprise cylindrical holes 38 and 39 which, with portions 30 and 31 coupled to each other, define the chamber 17 of the filter. As mentioned, the latter contains the ventilation filter 18 (cylindrical and per se known).

Furthermore, the second portion 31 has the threaded portion 20 of the hub which detaches or extends from a protruding flange 40. The vent hole 19 opens from one side 40A of such flange facing towards the portion 20. In particular, such hole opens at a radial recess 42 obtained in the flange 40 so as to define, with hub 2 coupled to the hollow cylindrical body 10, a radial channel 47 (generated when the flange 40 rests on the threaded part 21 of the body 10) through which the air ejected from the chamber 17 of the filter leaves the hypodermic needle 1.

As mentioned, should some blood drops exceed the ventilation filter 18, the blood would flow back (by capillary action) to the space 50 present between the threaded portion 20 of the hub 2 and the threaded part 21 of such body 10 and it would flow out from the radial channel 47.

Such through-flow of blood - if any - which flows from the hole 40 into the space 50 is also facilitated by a bevel 52 in the thread 20A of the threaded portion 20 which acts as a guide element for such through-flow of blood in said space 50. Alternatively, special capillary channels (not shown) may be provided on the contact surfaces between the constraint portion 20 and the constraint wall 21 should the type of coupling be other than the threaded one or in the event one wants to increase the effectiveness of the capillary action.

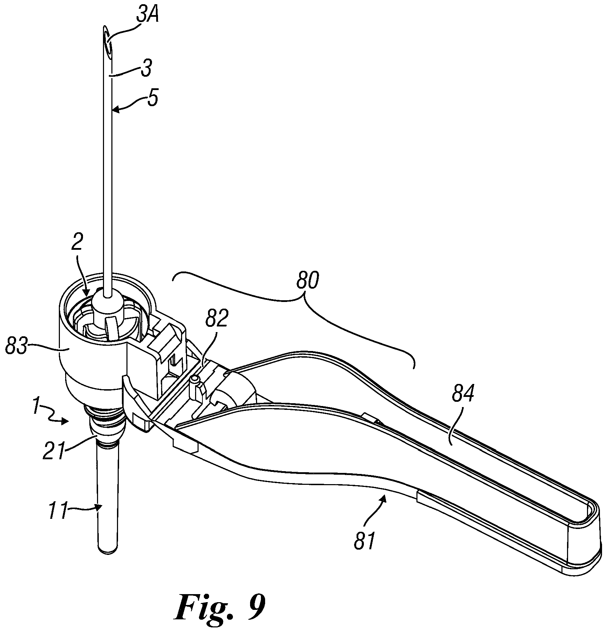

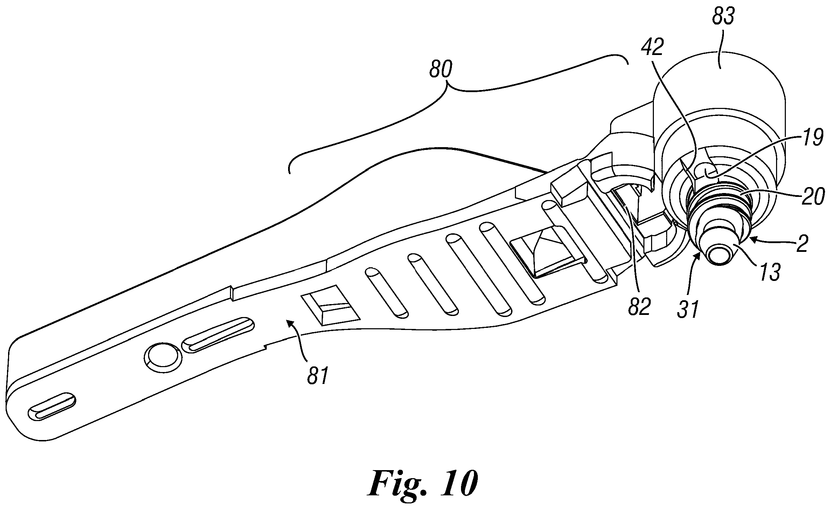

Figures 9 and 10, where parts corresponding to those of the figures already described previously are indicated using the same reference numerals, show a hypodermic needle 1 associated with a protection device 80 of the needle 5 adapted to prevent an operator collecting blood from subsequently coming into contact with the first portion 3 of the needle. Such device is for example of the type described in EP2750743 on behalf of the Applicant in question.

The protection device 80 comprises a protection arm 81 hinged in 82 to a connection element 83 adapted to be coupled to the hub 2 of the hypodermic needle 1 (for example by heat sealing) or the same defining a portion (such as the second portion 31 in figure 10) or the whole hub 2 of the hypodermic needle. In a known manner, after using the needle 5, the protection arm 81 may be rotated around the hinge 82 and brough above the first portion of the needle 5 which is received and blocked in a recess 84 of the aforementioned protection arm in a known manner.

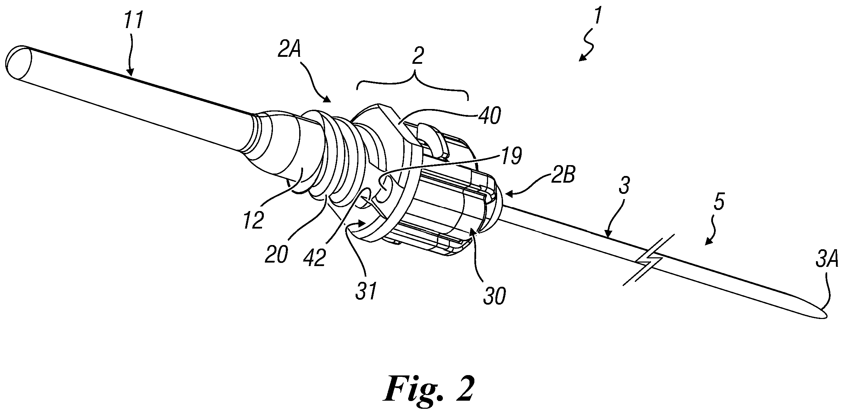

Also in the case of figures in question there is provided for the vent hole 19 arranged at the threaded portion 20 of the hub 2 so that ever outflow of blood - if any - can follow the thread 20A similar to what is described with reference to figures 1-8 (the thread 20A is always connected to a threaded part of a blood collection member or of a body adapted to contain member like the one indicated with 10 in the figures above) .

The invention allows to provide a hypodermic needle with characteristics adapted to allow air to be vented from the needle thereof when it is introduced into a vein and adapted to prevent or at least reduce to the maximum any blood spilling of the needle hub or support element thereof also acting as a reflux and display chamber. All this without jeopardising the size of the needle that can be used, the ease of manufacture of the hypodermic needle and the ease of use thereof.

Various embodiments of the invention have been described. However. others may also be possible without departing from the scope of protection of the invention, as defined by the claims that follow.

## Claims

1. A hypodermic needle (1) for multi-sample blood collection comprising a needle (5) having first and second portions (3, 4) projecting from opposite sides (2A, 2B) of a needle support element or hub (2) adapted to serve as a flashback chamber, the first portion (3) of the needle being adapted to the blood draw from a patient and the second portion (4) of the needle being adapted to cooperate with a blood collection element, the second portion (4) of the needle being covered by a covering element (11) adapted to be moved towards said needle support element (2) to uncover a tip (4A) of said second portion (4) when this second portion cooperates with the blood collection element, a space (15) being provided between said second portion (4) of the needle and the needle support element (2), said space (15) being adapted to allow the passage of the air present in the needle (5) towards an air vent hole (19) made in this needle support element (2), said air being pushed into a channel (6) of the needle by the blood drawn from the patient after the needle has been inserted thereof into a blood vessel of such patient, a ventilation filter (18) to retain any blood that should follow the air in its movement being provided along a path of said air from the channel (6) of the needle to the vent hole (19), wherein the vent hole (19) is being made with median longitudinal axis (K) parallel to that (W) of the needle (5), **characterised in that** said ventilation filter (18) is separated from the needle (5) and it is placed on one side thereof.

2. The hypodermic needle according to claim 1, **characterised in that** said needle support element (2) comprises a first and a second portion (30, 31), the second portion (4) of the needle coming out from the second portion (31), the second portion of the needle support element (2) having an external connecting portion (20), the vent hole (19) being close to this external connecting portion (20) and facing towards this external connecting portion (20).

3. The hypodermic needle according to claim 2, **characterised in that** said external connecting portion is alternatively an externally threaded portion (20) or a portion suitable to obtain an external connection by means of a bayonet coupling or snap-coupling.

4. The hypodermic needle according to claim 2, **characterised in that** said second portion (31) has a flange (40) from which the external connecting portion (20) departs, said vent hole (19) opening on one side (40A) of said flange facing said external connecting portion (20) of the needle support element (2).

5. The hypodermic needle according to claim 1, **characterised in that** the vent hole (19) is connected to a filter chamber (17) made in the needle support element (2) adapted to contain the ventilation filter (18).

6. The hypodermic needle according to claim 5, **characterised in that** said vent hole (19) is placed at one end of the chamber (17) of the filter containing the ventilation filter (18).

7. The hypodermic needle according to claim 5, **characterised in that** said filter chamber (17) has a longitudinal axis (K) parallel to that of the needle (5).

8. The hypodermic needle according to claim 4, **characterised in that** the side (40A) of the flange (40) in which the vent hole (19) opens has a radial depression (42) close to this vent hole (19).

9. A hypodermic needle and hollow cylindrical body assembly (10), said hypodermic needle (1) being according to claims 1 to 8, the hollow cylindrical body (10) being open at a first end (10A) and having the second end (10B) adapted to cooperate with said hypodermic needle (1), in particular by mechanical coupling of the external connecting portion (20) of the needle support element (2) of the hypodermic needle (1) with a coupling part (21) of the second end(10B) of said hollow cylindrical body (10), said needle support element (2) comprising a first and a second portion (30, 31), the second portion of the needle support element (2) having said external connecting portion (20), said second portion (31) having a flange (40) from which there departs the external connecting portion (20), said vent hole (19) opening on one side (40A) of said flange facing towards said external connecting portion (20) of the support element (2) of the needle, the side (40A) of the flange (40) in which the vent hole (19) opens having a radial depression (42) in proximity of such vent hole (19), **characterised in that** a radial channel (47) is provided between the hallow cylindrical body (10) and said flange (40), in said radial channel (47) opening the vent hole (19).

10. The assembly according to claim 9, **characterised in that** said external connecting portion (20) of the second portion (30) of the support element and said coupling part (21) of the hollow cylindrical body (10) are threaded, a fluid trap being defined between the thread of said portion (20) and part (21) connected each other.

11. The assembly according to claim 9, **characterised in that** said fluid trap is defined by discharge channels located between the first external connecting portion (20) of the second portion (30) of the support element (2) of the needle and the coupling part (21) of said hollow cylindrical body (10).

12. A hypodermic needle (1) and needle protection device (80) assembly, said hypodermic needle (1) being according to claims 1 to 8, said protection device (80) comprising a protection arm (81) hinged (in 82) to a connecting element (83) for connection to said hypodermic needle (1), said protection arm (81) comprising a recess (84) adapted to receive the first portion (3) of the needle (5) of said hypodermic needle (1) after use by the movement of said protection arm (81) around said hinge (82), **characterised in that** said connecting element (83) contains the support element (2) of the needle of the hypodermic needle (1), said support element being alternatively independent of said connecting element (83) or at least partly integral with said connecting element (83).

## Patentansprüche

1. Injektionsnadel (1) zum Mehrfachproben-Blutsammeln, umfassend eine Nadel (5), die einen ersten und einen zweiten Abschnitt (3, 4) aufweist, die von gegenüberliegenden Seiten (2A, 2B) eines Nadelträgerelements oder Nabe (2) hervorstehen, das/die angepasst ist, um als Rückflusskammer zu dienen, wobei der erste Abschnitt (3) der Nadel zum Entnehmen von Blut von einem Patienten angepasst ist und der zweite Abschnitt (4) der Nadel zum Zusammenwirken mit einem Blutsammelelement angepasst ist, der zweite Abschnitt (4) der Nadel durch ein Abdeckelement (11) abgedeckt ist, das angepasst ist, um in Richtung des Nadelträgerelements (2) bewegt zu werden, um eine Spitze (4A) des zweiten Abschnitts (4) freizulegen, wenn dieser zweite Abschnitt mit dem Blutsammelelement zusammenwirkt, ein Raum (15) zwischen dem zweiten Abschnitt (4) der Nadel und dem Nadelträgerelement (2) bereitgestellt ist, dieser Raum (15) angepasst ist, um den Durchgang der in der Nadel (5) vorhandenen Luft zu einem Entlüftungsloch (19) zu ermöglichen, das in diesem Nadelträgerelement (2) bereitgestellt ist, die Luft durch das Blut, das dem Patienten entnommen wird, in einen Kanal (6) der Nadel gedrückt wird, nachdem die Nadel in ein Blutgefäß des Patienten eingeführt worden ist, ein Belüftungsfilter (18), um jegliches Blut zurückzuhalten, das der Luft bei ihrer Bewegung folgen sollte, entlang eines Wegs der Luft von dem Kanal (6) der Nadel zu dem Entlüftungsloch (19) bereitgestellt ist, wobei das Entlüftungsloch (19) mit der mittleren Längsachse (K) parallel zu jener (W) der Nadel (5) angefertigt ist,
**dadurch gekennzeichnet, dass** der Belüftungsfilter (18) von der Nadel (5) getrennt und auf einer Seite davon angeordnet ist.

2. Injektionsnadel nach Anspruch 1, **dadurch gekennzeichnet, dass** das Nadelträgerelement (2) einen ersten und einen zweiten Abschnitt (30, 31) umfasst, wobei der zweite Abschnitt (4) der Nadel aus dem zweiten Abschnitt (31) hervorsteht, der zweite Abschnitt des Nadelträgerelements (2) einen äußeren Verbindungsabschnitt (20) aufweist, das Entlüftungsloch (19) in der Nähe dieses äußeren Verbindungsabschnitts (20) ist und diesem äußeren Verbindungsabschnitt (20) zugewandt ist.

3. Injektionsnadel nach Anspruch 2, **dadurch gekennzeichnet, dass** der äußere Verbindungsabschnitt alternativ ein Außengewindeabschnitt (20) oder ein Abschnitt ist, der geeignet ist, um eine äußere Verbindung mittels einer Bajonettkupplung oder einer Einrastkupplung zu erlangen.

4. Injektionsnadel nach Anspruch 2, **dadurch gekennzeichnet, dass** der zweite Abschnitt (31) einen Flansch (40) aufweist, von dem der äußere Verbindungsabschnitt (20) ausgeht, wobei das Entlüftungsloch (19) auf einer Seite (40A) des Flanschs mündet, die dem äußeren Verbindungsabschnitt (20) des Nadelträgerelements (2) zugewandt ist.

5. Injektionsnadel nach Anspruch 1, **dadurch gekennzeichnet, dass** das Entlüftungsloch (19) mit einer Filterkammer (17) verbunden ist, die in dem Nadelträgerelement (2) angefertigt und angepasst ist, um den Belüftungsfilter (18) aufzunehmen.

6. Injektionsnadel nach Anspruch 5, **dadurch gekennzeichnet, dass** die Entlüftungsöffnung (19) an einem Ende der Kammer (17) des Filters platziert ist, die den Belüftungsfilter (18) enthält.

7. Injektionsnadel nach Anspruch 5, **dadurch gekennzeichnet, dass** die Filterkammer (17) eine Längsachse (K) aufweist, die parallel zu jener der Nadel (5) ist.

8. Injektionsnadel nach Anspruch 4, **dadurch gekennzeichnet, dass** die Seite (40A) des Flanschs (40), in die das Entlüftungsloch (19) mündet, eine radiale Vertiefung (42) in der Nähe dieses Entlüftungslochs (19) aufweist.

9. Injektionsnadel- und Hohlzylinderkörperanordnung (10), wobei die Injektionsnadel (1) nach einem der Ansprüche 1 bis 8 ist, der Hohlzylinderkörper (10) an einem ersten Ende (10A) offen ist und das zweite Ende (10B) aufweist, das angepasst ist, um mit der Injektionsnadel (1) zusammenzuwirken, insbesondere durch mechanisches Kuppeln des äußeren Verbindungsabschnitts (20) des Nadelträgerelements (2) der Injektionsnadel (1) mit einem Kupplungsteil (21) des zweiten Endes (10B) des Hohlzylinderkörpers (10), das Nadelträgerelement (2) umfassend einen ersten und einen zweiten Abschnitt (30, 31), wobei der zweite Abschnitt des Nadelträgerelements (2) den äußeren Verbindungsabschnitt (20) aufweist, der zweite Abschnitt (31) einen Flansch (40) aufweist, von dem der äußere Verbindungsabschnitt (20) ausgeht, das Entlüftungsloch (19) auf einer Seite (40A) des Flansches mündet, die dem äußeren Verbindungsabschnitt (20) des Trägerelements (2) der Nadel zugewandt ist, die Seite (40A) des Flanschs (40), in die das Entlüftungsloch (19) mündet, eine radiale Vertiefung (42) in der Nähe dieses Entlüftungslochs (19) aufweist, **dadurch gekennzeichnet, dass** ein radialer Kanal (47) zwischen dem Hohlzylinderkörper (10) und dem Flansch (40) bereitgestellt ist, wobei das Entlüftungsloch (19) in den radialen Kanal (47) mündet.

10. Anordnung nach Anspruch 9, **dadurch gekennzeichnet, dass** der äußere Verbindungsabschnitt (20) des zweiten Abschnitts (30) des Trägerelements und der Kupplungsteil (21) des Hohlzylinderkörpers (10) mit Gewinde versehen sind, wobei eine Fluidfalle zwischen dem Gewinde des Abschnitts (20) und dem Teil (21), die miteinander verbunden sind, definiert ist.

11. Anordnung nach Anspruch 9, **dadurch gekennzeichnet, dass** die Fluidfalle durch Abflusskanäle definiert ist, die sich zwischen dem ersten äußeren Verbindungsabschnitt (20) des zweiten Abschnitts (30) des Trägerelements (2) der Nadel und dem Kupplungsteil (21) des Hohlzylinderkörpers (10) befinden.

12. Anordnung aus Injektionsnadel (1) und Nadelschutzvorrichtung (80), wobei die Injektionsnadel (1) nach einem der Ansprüche 1 bis 8 ist, die Schutzvorrichtung (80) umfassend einen Schutzarm (81), der an ein Verbindungselement (83) zur Verbindung mit der Injektionsnadel (1) angelenkt (in 82) ist, wobei der Schutzarm (81) eine Aussparung (84) aufweist, die angepasst ist, um den ersten Abschnitt (3) der Nadel (5) der Injektionsnadel (1) nach Benutzung durch die Bewegung des Schutzarms (81) um das Gelenk (82) aufzunehmen, **dadurch gekennzeichnet, dass** das Verbindungselement (83) das Trägerelement (2) der Nadel der Injektionsnadel (1) enthält, wobei das Trägerelement alternativ unabhängig von dem Verbindungselement (83) oder zumindest teilweise mit dem Verbindungselement (83) verbunden ist.

## Revendications

1. Aiguille hypodermique (1) destinée au prélèvement de plusieurs échantillons de sang, comprenant une aiguille (5) dotée d'une première et d'une deuxième parties (3, 4) faisant saillie à partir des côtés opposés (2A, 2B) d'un élément de support d'aiguille ou embase (2) adapté pour servir de chambre de refoulement, la première partie (3) de l'aiguille étant adaptée pour prélever le sang d'un patient et la deuxième partie (4) de l'aiguille étant adaptée pour coopérer avec un élément de prélèvement de sang, la deuxième partie (4) de l'aiguille étant recouverte par un élément de recouvrement (11) adapté pour être déplacé vers ledit élément de support d'aiguille (2) afin de découvrir une pointe (4A) de ladite deuxième partie (4) lorsque cette deuxième partie coopère avec l'élément de prélèvement de sang, un espace (15) étant prévu entre ladite deuxième partie (4) de l'aiguille et l'élément de support d'aiguille (2), ledit espace (15) étant adapté pour permettre le passage de l'air présent dans l'aiguille (5) vers un orifice d'aération (19) pratiqué dans cet élément de support d'aiguille (2), ledit air étant poussé dans un canal (6) de l'aiguille par le sang prélevé du patient après que l'aiguille ait été insérée dans un vaisseau sanguin dudit patient, un filtre de ventilation (18) destiné à retenir tout sang qui pourrait suivre l'air dans son mouvement prévu le long d'un trajet dudit air depuis le canal (6) de l'aiguille à l'orifice d'aération (19), l'orifice d'aération (19) étant réalisé avec un axe longitudinal médian (K) parallèle à celui (W) de l'aiguille (5), **caractérisé en ce que** ledit filtre de ventilation (18) est séparé de l'aiguille (5) et placé sur un côté de celle-ci.

2. Aiguille hypodermique selon la revendication 1, **caractérisée en ce que** ledit élément de support d'aiguille (2) comprend une première et une deuxième partie (30, 31), la deuxième partie (4) de l'aiguille sortant de la deuxième partie (31), la deuxième partie de l'élément de support d'aiguille (2) comportant une partie de raccordement externe (20), l'orifice d'aération (19) étant proche de cette partie de raccordement externe (20) et faisant face à cette partie de raccordement externe (20).

3. Aiguille hypodermique selon la revendication 2, **caractérisée en ce que** ladite partie de raccordement externe est soit une partie filetée à l'extérieur (20), soit une partie permettant d'obtenir un raccordement externe au moyen d'un raccord à baïonnette ou d'un raccord à encliquetage.

4. Aiguille hypodermique selon la revendication 2, **caractérisée en ce que** ladite deuxième partie (31) comporte une bride (40) de laquelle part la partie de raccordement externe (20), ledit orifice d'aération (19) s'ouvrant sur un côté (40A) de ladite bride faisant face à ladite partie de raccordement externe (20) de l'élément de support d'aiguille (2).

5. Aiguille hypodermique selon la revendication 1, **caractérisée en ce que** l'orifice d'aération (19) est raccordé à une chambre de filtration (17) réalisée dans l'élément de support d'aiguille (2) adapté pour contenir le filtre de ventilation (18).

6. Aiguille hypodermique selon la revendication 5, **caractérisée en ce que** ledit orifice d'aération (19) est placé à une extrémité de la chambre (17) du filtre contenant le filtre de ventilation (18).

7. Aiguille hypodermique selon la revendication 5, **caractérisée en ce que** ladite chambre de filtration (17) présente un axe longitudinal (K) parallèle à celui de l'aiguille (5).

8. Aiguille hypodermique selon la revendication 4, **caractérisée en ce que** le côté (40A) de la bride (40) dans lequel s'ouvre l'orifice d'aération (19) présente une dépression radiale (42) proche de cet orifice d'aération (19).

9. Ensemble formé d'une aiguille hypodermique et d'un corps cylindrique creux (10), ladite aiguille hypodermique (1) étant conforme aux revendications 1 à 8, le corps cylindrique creux (10) étant ouvert à une première extrémité (10A) et sa deuxième extrémité (10B) étant adaptée pour coopérer avec ladite aiguille hypodermique (1), en particulier par couplage mécanique de la partie de raccordement externe (20) de l'élément de support d'aiguille (2) de l'aiguille hypodermique (1) avec une partie de couplage (21) de la deuxième extrémité (10B) dudit corps cylindrique creux (10), ledit élément de support d'aiguille (2) comprenant une première et une deuxième parties (30, 31), la deuxième partie de l'élément de support d'aiguille (2) comportant ladite partie de connexion externe (20), ladite deuxième partie (31) comportant une bride (40) de laquelle part la partie de raccordement externe (20), ledit orifice d'aération (19) s'ouvrant sur un côté (40A) de ladite bride orientée vers ladite partie de raccordement externe (20) de l'élément de support (2) de l'aiguille, le côté (40A) de la bride (40) dans lequel s'ouvre l'orifice d'aération (19) comportant une dépression radiale (42) à proximité dudit orifice d'aération (19), **caractérisé en ce qu'**un canal radial (47) est prévu entre le corps cylindrique creux (10) et ladite bride (40), dans ledit canal radial (47) en ouvrant l'orifice d'aération (19).

10. Ensemble selon la revendication 9, **caractérisé en ce que** ladite partie de raccordement externe (20) de la deuxième partie (30) de l'élément de support et ladite partie d'accouplement (21) du corps cylindrique creux (10) sont filetées, un piège à fluide étant défini entre le filetage de ladite partie (20) et la partie (21) raccordées l'une à l'autre.

11. Ensemble selon la revendication 9, **caractérisé en ce que** ledit piège à fluide est défini par des canaux d'évacuation situés entre la première partie de raccordement externe (20) de la deuxième partie (30) de l'élément de support (2) d'aiguille et la partie de raccordement (21) dudit corps cylindrique creux (10).

12. Ensemble formé d'une aiguille hypodermique (1) et d'un dispositif de protection d'aiguille (80), ladite aiguille hypodermique (1) étant conforme aux revendications 1 à 8, ledit dispositif de protection (80) comprenant un bras de protection (81) articulé (en 82) à un élément de raccordement (83) destiné à être raccordé à ladite aiguille hypodermique (1), ledit bras de protection (81) comprenant un évidement (84) adapté pour recevoir la première partie (3) de l'aiguille (5) de ladite aiguille hypodermique (1) après utilisation par le mouvement dudit bras de protection (81) autour dudit pivot (82), **caractérisé en ce que** ledit élément de connexion (83) contient l'élément de support (2) d'aiguille de l'aiguille hypodermique (1), ledit élément de support étant soit indépendant dudit élément de raccordement (83), soit au moins en partie fixé audit élément de raccordement (83).
